## Europäisches Patentamt

(19) **European Patent Office**

**Office européen des brevets**

(11) Publication number: **0 137 689**

**B1**

(12) # EUROPEAN PATENT SPECIFICATION

(45) Date of publication of patent specification: **10.12.86**

(51) Int. Cl.⁴: **C 07 K 7/10, A 61 K 37/02, A 61 K 37/24**

(21) Application number: **84305845.4**

(22) Date of filing: **28.08.84**

(54) GRF analogs.

(30) Priority: **29.08.83 US 527292**
**12.10.83 US 541167**
**02.03.84 US 585814**

(43) Date of publication of application:
**17.04.85 Bulletin 85/16**

(45) Publication of the grant of the patent:
**10.12.86 Bulletin 86/50**

(84) Designated Contracting States:
**AT BE CH DE FR GB IT LI LU NL SE**

(56) References cited:
**EP-A-0 107 890**
**EP-A-0 117 034**

**PROCEEDINGS OF THE NATIONAL ACADEMY OF SCIENCES, vol. 80, 1983 U. GUBLER et al. "Cloning and sequence analysis of cDNA for the precursor of human growth hormone releasing factor, somatocrinin" pages 4311-4314**

**NATURE, vol. 303, 1983, J. SPIESS et al. "Characterization of rat hypothalamic growth hormono-releasing factor", pages 532-535**

(73) Proprietor: **THE SALK INSTITUTE FOR BIOLOGICAL STUDIES**
**10010 North Torrey Pines Road**
**La Jolla California 92037 (US)**

(72) Inventor: **Bohlen, Peter, Prof.**
**147 Honeycomb Court**
**Encinitas California 92024 (US)**
Inventor: **Brazeau, Paul Ernest**
**Rue 95 McNider No. 204**
**Outremont Quebec H2V 3X5 (CA)**
Inventor: **Esch, Frederick Stephen**
**2929 Fire Mountain Drive No. 69**
**Oceanside California 92054 (US)**
Inventor: **Ling, Nicholas Chai-Kwan, Prof.**
**5324 Bloch Street**
**San Diego California 92122 (US)**
Inventor: **Wehrenberg, William Busse, Prof.**
**5811 Tulane Street**
**San Diego California 92122 (US)**
Inventor: **Guillemin, Roger Charles Louis, Prof.**
**7316 Encelia Drive**
**La Jolla California 92037 (US)**

(74) Representative: **Lawrence, Malcolm Graham et al**
**Malcolm Lawrence & Co. 9th Floor Terminus House Terminus Street**
**Harlow Essex CM20 1XF (GB)**

**0 137 689**

56 References cited:
BIOCHEMICAL AND BIOPHYSICAL RESEARCH
COMMUNICATIONS, vol. 109, 1982 F.S. EASCH
et al. "Characterization of a 40 residue peptide
from a human pancreatic tumor with growth
hormone releasing activity" pages 152-158

## Description

The present invention relates to peptides having influence on the function of the pituitary gland in mammals. In particular, the present invention is directed to peptides which promote the release of growth hormone by the pituitary gland and which are particularly useful with respect to particular species.

In 1982, a hypothalamic releasing factor for pituitary growth hormone or somatotropin was isolated from a human islet cell tumor, purified, characterized and synthesized. When tested, it was found to promote the release of growth hormone (GH) by the pituitary. This peptide has the sequence:

H-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-Gln-Gln-Gly-Glu-Ser-Asn-Gln-Glu-Arg-Gly-Ala-Arg-Ala-Arg-Leu-NH$_2$.

Human hypothalamic growth hormone releasing factor (hGRF) has now been found to have the same structure, Bohlen et. al. *Biochem. and Biophs. Res. Comm.*, **114**, 3, pp. 930—936 (1983).

Now 44-residue polypeptides have been isolated from purified extracts of porcine, bovine, caprine and ovine hypothalami and characterized. They were each found to have a difference of a few amino acid residues from the formula of hGRF as set forth hereinafter.

In terms of the composition of hGRF, Porcine GRF (pGRF) may be expressed as the analog [Arg$^{34}$, Gln$^{38}$, Val$^{42}$]-hGRF(1—44)-NH$_2$ which means it has the formula:

H-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-Gln-Gln-Gly-Glu-Arg-Asn-Gln-Glu-Gln-Gly-Ala-Arg-Val-Arg-Leu-NH$_2$.

It is hereinafter referred to as pGRF or porcine somatocrinin. This peptide can be used to promote growth of warm-blooded animals, particularly hogs, of cold-blooded animals and in agriculture.

In terms of the composition of hGRF, bovine GRF (bGRF) may be expressed as the analog [Asn$^{28}$, Arg$^{34}$, Gln$^{38}$, Lys$^{41}$, Val$^{42}$]-hGRF (1—44)-NH$_2$ which means it has the formula:

H-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Asn-Arg-Gln-Gln-Gly-Glu-Arg-Asn-Gln-Glu-Gln-Gly-Ala-Lys-Val-Arg-Leu-NH$_2$.

It is hereinafter referred to as bGRF or bovine somatocrinin. Caprine GRF has the same formula and can be used to promote growth of warm-blooded animals, particularly cattle, of cold-blooded animals and in agriculture. It may also be used to increase milk production in cows and in goats in order to provide milk for making speciality cheeses.

In terms of the composition of hGRF, ovine GRF (oGRF) may be expressed as the analog [Ile$^{13}$, Asn$^{28}$, Arg$^{34}$, Gln$^{38}$, Lys$^{41}$, Val$^{42}$]-hGRF (1—44)-NH$_2$ which means it has the formula:

H-Tyr-Ala-Asp-Ala-Ile-Phe-Thr-Asn-Ser-Tyr-Arg-Lys-Ile-Leu-Gly-Gln-Leu-Ser-Ala-Arg-Lys-Leu-Leu-Gln-Asp-Ile-Met-Asn-Arg-Gln-Gln-Gly-Glu-Arg-Asn-Gln-Glu-Gln-Gly-Ala-Lys-Val-Arg-Leu-NH$_2$.

It is hereinafter referred to as oGRF or ovine somatocrinin. This peptide can be used to promote growth of warm-blooded animals, particularly sheep, of cold-blooded animals and in aquiculture. It may also be used to increase milk production in ewes in order to provide milk for making specialty cheeses.

Veterinary and pharmaceutical compositions in accordance with the invention include either pGRF, bGRF or oGRF, an analog thereof or a biologically active fragment thereof, or a nontoxic salt of any of the foregoing, dispersed in a pharmaceutically or veterinarily acceptable liquid of solid carrier. Such compositions can be used in clinical medicine, both human and veterinary, in acute or chronic administration for diagnostic or therapeutic purposes. Moreover, they can be used to accelerate the growth of muscle mass and/or milk production in hogs, cattle, goats, sheep or other animals.

The nomenclature used to define the peptides is that specified by Schroder & Lubke, "The Peptides", Academic Press (1965), wherein in accordance with conventional representation the amino group at the N-terminal appears to the left and the carboxyl group at the C-terminal to the right. Where the amino acid residue has isomeric forms, it is the L-form of the amino acid that is represented unless otherwise expressly indicated.

The invention provides synthetic GRF peptides having the following formula:

H-Tyr-Ala-Asp-Ala-Ile-Phe-Thr-Asn-Ser-Tyr-Arg-Lys-R$_{13}$-Leu-Gly-Gln-Leu-Ser-Ala-Arg-Lys-Leu-Leu-Gln-Asp-Ile-Met-R$_{28}$-Arg-Gln-Gln-Gly-Glu-Arg-Asn-Gln-Glu-Gln-Gly-Ala-R$_{41}$-Val-Arg-Leu-Y

wherein R$_{13}$ is Val or Ile; R$_{28}$ is Ser or Asn; R$_{41}$ is Arg or Lys; and Y is OH or NH$_2$. Also included are biologically active fragments containing the N-terminal segment (eg. fragments extending from the N-terminus to at least about residue-28 (or to at least about residue-34 for pGRF)) where Y can be either OH or NH$_2$.

The peptides can be synthesized by any suitable method, such as by exclusively solid-phase

3

techniques, by partial solid-phase techniques, by fragment condensation, by classical solution couplings, or by the employment of recently developed recombinant DNA techniques. For example, the techniques of exclusively solid-phase synthesis are set forth in the textbook "Solid-Phase Peptide Synthesis", Stewart & Young, Freeman & Co., San Francisco, 1969, and are exemplified by the disclosure of U.S. Patent No. 4,105,603, issued August 8, 1978. The fragment condensation method of synthesis is exemplified in U.S. Patent No. 3,972,859 (August 3, 1976). Other available syntheses are exemplified by U.S. Patent No. 3,842,067 (October 15, 1974) and U.S. Patent No. 3,862,925 (January 28, 1975). Production of the synthetic peptides using recombinant DNA techniques will likely be used to satisfy large-scale commercial requirements.

Synthesis by the use of recombinant DNA techniques, for purposes of this application, should be understood to include the suitable employment of a structural gene coding for a form of GRF. The synthetic GRF may be obtained by transforming a microorganism using an expression vector including a promoter and operator together with such structural gene and causing such transformed microorganism to express GRF. A non-human animal may also be used to produce GRF by gene-farming using such a structural gene and the general techniques set forth in U.S. Patent No. 4,276,282 issued June 30, 1981 or using microinjection of embryos as described in WO83/01783 published 26 May 1983 and WO82/04443 published 23 December 1982. The synthetic GRF may also be produced directly in the animal for which accelerated growth is intended by the techniques described in the two WO publications.

Common to coupling-type syntheses is the protection of the labile side chain groups of the various amino acid moieties with suitable protecting groups which will prevent a chemical reaction from occurring at that site until the group is ultimately removed. Usually also common is the protection of an alpha-amino group on an amino acid or a fragment while that entity reacts at the carboxyl group, followed by the selective removal of the alpha-amino protecting group to allow subsequent reaction to take place at that location. Accordingly, it is common that, as a step in the synthesis, an intermediate compound is produced which includes each of the amino acid residues located in its desired sequence in the peptide chain with side-chain protecting groups linked to the appropriate residues.

Also considered to be within the scope of the present invention are intermediates of the formula:

$$X^1\text{-Tyr}(X^2)\text{-Ala-Asp}(X^3)\text{-Ala-Ile-Phe-Thr}(X^4)\text{-Asn-Ser}(X^5)\text{-Tyr}(X^2)\text{-Arg}(X^6)\text{-Lys}(X^7)\text{-}R_{13}\text{-Leu-Gly-Gln-Leu-}$$
$$\text{Ser}(X^5)\text{-Ala-Arg}(X^6)\text{-Lys}(X^7)\text{-Leu-Leu-Gln-Asp}(X^3)\text{-Ile-Met-}R_{28}(X^5)\text{-Arg}(X^6)\text{-Gln-Gln-Gly-Glu}(X^3)\text{-Arg}(X^6)\text{-Asn-}$$
$$\text{Gln-Glu}(X^3)\text{-Gln-Gly-Ala-}R_{41}(X^6 \text{ or } X^7)\text{-Val-Arg}(X^6)\text{-Leu-}X^8$$

wherein: $X^1$ is either hydrogen or an α-amino protecting group. The α-amino protecting groups contemplated by $X^1$ are those known to be useful in the art of step-wise synthesis of polypeptides. Among the classes of α-amino protecting groups covered by $X^1$ are (1) acyl-type protecting groups, such as formyl, trifluoroacetyl, phthalyl, toluenesulfonyl(Tos), benzenesulfonyl, nitrophenylsulfenyl, tritylsulfenyl, o-nitrophenoxyacetyl, chloroacetyl, acetyl, and α-chlorobutyryl; (2) aromatic urethan-type protecting groups, such as benzyloxycarbonyl (Z) and substituted Z, such as p-chlorobenzyloxycarbonyl, p-nitrobenzyloxycarbonyl, p-bromobenzyloxycarbonyl, p-methoxybenzyloxycarbonyl; (3) aliphatic urethan protecting groups, such as t-butyloxycarbonyl (BOC), diisopropylmethyloxycarbonyl, isopropyloxycarbonyl, ethoxycarbonyl, allyloxycarbonyl; (4) cycloalkyl urethan-type protecting groups, such as cyclopentyloxycarbonyl, adamantyloxycarbonyl, and cyclohexyloxycarbonyl; (5) thiourethan-type protecting groups, such as phenylthiocarbonyl; (6) alkyl-type protecting groups, such as triphenylmethyl (trityl), benzyl; (7) trialkylsilane groups, such as trimethylsilane. The preferred α-amino protecting group is BOC.

$X^2$ is a protecting group for the phenolic hydroxyl group of Tyr selected from the group consisting of tetrahydropyranyl, tert-butyl, trityl, Bzl, CBZ, 4Br-CBZ and 2,6-dichlorobenzyl. The preferred protecting group is 2,6-dichlorobenzyl. $X^2$ can be hydrogen which means that there is no protecting group on the hydroxyl group.

$X^3$ is hydrogen or an ester-forming protecting group for the carboxyl group of Asp or Glu and is selected from the group consisting of Bzl, 2,6-dichlorobenzyl, methyl and ethyl.

$X^4$ and $X^5$ are protecting groups for the hydroxyl group of Thr and Ser and are selected from the group consisting of acetyl, benzoyl, tert-butyl, trityl, tetrahydropyranyl, Bzl, 2,6-dichlorobenzyl and CBZ. The preferred protecting group is Bzl. $X^4$ and/or $X^5$ can be hydrogen, which means there is no protecting group on the hydroxyl group.

$X^6$ is a protecting group for the guanidino group of Arg selected from the group consisting of nitro, Tos, CBZ, adamantyloxycarbonyl, and BOC, or is hydrogen;

$X^7$ is hydrogen or a protecting group for the side chain amino substituent of Lys. Illustrative of suitable side chain amino protecting groups are 2-chlorobenzyloxycarbonyl (2-Cl-Z), Tos, CBZ, t-amyloxycarbonyl and BOC.

The selection of a side chain amino protecting group is not critical except that it must be one which is not removed during deprotection of the α-amino groups during the synthesis. Hence, the α-amino protecting group and the side chain amino protecting group cannot be the same.

Optionally the side chain amido group of Gln and/or Asn can be suitably protected as with xanthyl (Xan).

4

$X^8$ is selected, from the class consisting of OH, OCH$_3$, esters, amides, hydrazides, —O—CH$_2$-resin support and —NH-resin support, with the groups other than OH and amides being broadly considered as protecting groups.

In the formula for the intermediate, at least one of $X^1$, $X^2$, $X^3$, $X^4$, $X^5$, $X^6$, $X^7$ and $X^8$ is a protecting group.

In selecting a particular side chain protecting group to be used in the synthesis of the peptides, the following rules are followed: (a) the protecting group should be stable to the reagent and under the reaction conditions selected for removing the α-amino protecting group at each step of the synthesis, (b) the protecting group should retain its protecting properties and not be split off under coupling conditions, and (c) the side chain protecting group should be removable, upon the completion of the synthesis containing the desired amino acid sequence, under reaction conditions that will not alter the peptide chain.

The peptides are preferably prepared using solid phase synthesis, such as that described by Merrifield, *J. Am. Chem. Soc.,* 85, p 2149 (1963), although other equivalent chemical syntheses known in the art can also be used as previously mentioned. Solid-phase synthesis is commenced from the C-terminal end of the peptide by coupling a protected α-amino acid to a suitable resin. Such a starting material can be prepared by attaching α-amino-protected Leu by an ester linkage to a chloromethylated resin or a hydroxymethyl resin, or by an amide bond to a BHA resin or MBHA resin. The preparation of the hydroxymethyl resin is described by Bodansky et al., *Chem. Ind.* (London) 38, 1597—98 (1966). Chloromethylated resins are commercially available from Bio Rad Laboratories, Richmond, California and from Lab. Systems, Inc. The preparation of such a resin is described by Stewart et al., "Solid Phase Peptide Synthesis" (Freeman & Co., San Francisco 1969), Chapter 1, pp 1—6. BHA and MBHA resin supports are commercially available and are generally used only when the desired polypeptide being synthesized has an α-carboxamide at the C-terminal.

Leu protected by BOC is coupled to the chloromethylated resin according to the procedure of Monahan and Gilon, *Biopolymer* 12, pp 2513—19, 1973 when, for example, it is desired to synthesize the 44-amino acid peptide with free carboxy terminal. Following the coupling of BOC-Leu, the α-amino protecting group is removed, as by using trifluoroacetic acid (TFA) in methylene chloride, TFA alone or HCl in dioxane. The deprotection is carried out at a temperature between about 0°C and room temperature. Other standard cleaving reagents and conditions for removal of specific α-amino protecting groups may be used as described in Schroder & Lubke, "The Peptides", 1 pp 72—75 (Academic Press 1965).

After removal of the α-amino protecting group of Leu, the remaining α-amino- and side chain-protected amino acids are coupled step-wise in the desired order to obtain the intermediate compound defined hereinbefore, or as an alternative to adding each amino acid separately in the synthesis, some of them may be coupled to one another prior to addition to the solid phase reactor. The selection of an appropriate coupling reagent is within the skill of the art. Particularly suitable as a coupling reagent is N,N'-dicyclohexyl carbodiimide (DCCI).

The activating reagents used in the solid phase synthesis of the peptides are well known in the peptide art. Examples of suitable activating reagents are: (1) carbodiimides, such as N,N'-diisopropyl carbodiimide, N-N'-dicyclohexylcarbodiimide (DCCI); (2) cyanamides such as N,N'-dibenzylcyanamide; (3) keteimines; (4) isoxazolium salts, such as N-ethyl-5-phenyl isoxazolium-3'-sulfonate; (5) monocyclic nitrogen-containing heterocyclic amides of aromatic character containing one through four nitrogens in the ring, such as imidazolides, pyrazolides, and 1,2,4-triazolides. Specific heterocyclic amides that are useful include N,N'-carbonyl dimidazole, N,N'-carbonyl-di-1,2,4-triazole; (6) alkoxylated acetylene, such as ethoxyacetylene; (7) reagents which form a mixed anhydride with the carboxyl moiety of the amino acid, such as ethylchloroformate and isobutylchloroformate and (8) reagents which form an active ester with the carboxyl moiety of the amino acid, such as nitrogen-containing heterocyclic compounds having a hydroxy group on one ring nitrogen, e.g. N-hydroxyphthalimide, N-hydroxysuccinimide and 1-hydroxybenzotriazole (HOBT). Other activating reagents and their use in peptide coupling are described by Schroder & Lubke supra, in Chapter III and by Kapoor, *J. Phar. Sci.,* 59, pp 1—27 (1970).

Each protected amino acid or amino acid sequence is introduced into the solid phase reactor in about a twofold or more excess, and the coupling may be carried out in a medium of dimethylformamide (DMF): CH$_2$Cl$_2$ (1:1) or in DMF or CH$_2$Cl$_2$ alone. In cases where incomplete coupling occurred, the coupling procedure is repeated before removal of the α-amino protecting group prior to the coupling of the next amino acid. The success of the coupling reaction at each stage of the synthesis is monitored by the ninhydrin reaction, as described by E. Kaiser et al., *Anal. Biochem.* 34, 595 (1970).

After the desired amino acid sequence has been completed, the intermediate peptide is removed from the resin support by treatment with a reagent, such as liquid hydrogen fluoride, which not only cleaves the peptide from the resin but also cleaves all remaining side chain protecting groups $X^2$, $X^3$, $X^4$, $X^5$, $X^6$, $X^7$ and $X^8$ and the α-amino protecting group $X^1$, to obtain the peptide.

As an alternative route, the intermediate peptide may be separated from the resin support by alcoholysis after which the recovered C-terminal alkyl ester is converted to the acid by hydrolysis. Any side chain protecting groups may then be cleaved as previously described or by other known procedures, such as catalytic reduction (e.g. Pd on BaSO$_4$). When using hydrogen fluoride for cleaving, anisole and methylethyl sulfide are included in the reaction vessel for scavenging.

The following Examples set forth preferred methods for synthesizing GRF by the solid-phase technique.

**0 137 689**

Example 1
The synthesis of pGRF (1—44) free acid having the formula:

H-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-
Asp-Ile-Met-Ser-Arg-Gln-Gln-Gly-Glu-Arg-Asn-Gln-Glu-Gln-Gly-Ala-Arg-Val-Arg-Leu-OH

is conducted in a stepwise manner using a Beckman 990 Peptide Synthesizer on a chloromethylated resin, such as that available from Lab Systems, Inc., containing 0.9 Meq Cl/g. Coupling of BOC-Leu to the resin is performed by the general procedure set forth by Monahan et al. in *Biopolymers,* Volume 12 (1973) pp. 2513—2519, and it results in the substitution of about 0.22 mmol. Leu per gram of resin. All solvents that are used are carefully degassed by sparging with an inert gas, preferably helium, to insure the absence of oxygen that might undesirably oxidize the sulfur of the Met residue.

After deprotection and neutralization, the peptide chain is built step-by-step on the resin. Deprotection, neutralization and addition of each amino acid is performed in general accordance with the procedure set forth in detail in Guillemin et al. U.S. Patent No. 3,904,594. The couplings are specifically carried out as set out in the following Schedule A:

**0 137 689**

SCHEDULE

| STEP | REAGENTS AND OPERATIONS | MIX TIMES MIN. |
|---|---|---|
| 1 | $CH_2Cl_2$ wash (2 times) | 0.5 |
| 2 | 50% trifluoroacetic acid (TFA) + 5% 1,2-ethanedithiol in $CH_2Cl_2$ (1 time) | 0.5 |
| 3 | 50% trifluoroacetic acid (TFA) + 5% 1,2-ethanedithiol in $CH_2Cl_2$ (1 time) | 20.0 |
| 4 | $CH_2Cl_2$ wash (3 times) | 0.5 |
| 5 | $CH_3OH$ wash (2 times) | 0.5 |
| 6 | 10% triethylamine ($Et_3N$) in $CH_2Cl_2$ neutralization (2 times) | 0.5 |
| 7 | $CH_3OH$ wash (2 times) | 0.5 |
| 8 | 10% triethylamine ($Et_3N$) in $CH_2Cl_2$ neutralization (2 times) | 0.5 |
| 9 | $CH_3OH$ wash (2 times) | 0.5 |
| 10 | $CH_2Cl_2$ wash (2 times) | 0.5 |
| 11 | *Boc-amino acid (1 mmole/g resin) plus equivalent amount of dicyclohexylcarbodiimmide (DCC) in $CH_2Cl_2$ | 120 |
| 12 | $CH_2Cl_2$ wash (1 time) | 0.5 |
| 13 | 50% dimethylformamide in $CH_2Cl_2$ wash (2 times) | 0.5 |
| 14 | 10% triethylamine ($Et_3N$) in $CH_2Cl_2$ wash (1 time) | 0.5 |
| 15 | $CH_3OH$ wash (2 times) | 0.5 |
| 16 | $CH_2Cl_2$ wash (2 times) | 0.5 |
| 17 | 25% acetic anhydride in $CH_2Cl_2$ (2 ml/g resin) | 20.0 |
| 18 | $CH_2Cl_2$ wash (2 times) | 0.5 |
| 19 | $CH_3OH$ wash (2 times) | 0.5 |

*For the coupling of Asn and Gln, an 1.136 molar excess of 1-hydroxybenzotriazole (HOBt) was included in this step.

Briefly, for the coupling reaction, one mmol. of BOC-protected amino acid in methylene chloride is used per gram of resin, plus one equivalent of 0.5 molar DCCI in methylene chloride or 30% DMF in methylene chloride, for two hours. When Arg is being coupled, a mixture of 10% DMF and methylene chloride is used. Bzl is used as the hydroxyl side-chain protecting group for Ser and Thr. 2-chloro-benzyloxycarbonyl (2Cl-Z) is used as the protecting group for the Lys side chain. Tos is used to protect the guanidino group of Arg, and the Glu or Asp carboxyl group is protected as the Bzl ester. The phenolic hydroxyl group of Tyr is protected with 2,6-dichlorobenzyl. At the end of the synthesis, the following composition is obtained:

7

$X^1$-Tyr($X^2$)-Ala-Asp($X^3$)-Ala-Ile-Phe-Thr($X^4$)-Asn-Ser($X^5$)-Tyr($X^2$)-Arg($X^6$)-Lys($X^7$)-Val-Leu-Gly-Gln-Leu-Ser($X^5$)-Ala-Arg($X^6$)-Lys($X^7$)-Leu-Leu-Gln-Asp($X^3$)-Ile-Met-Ser($X^5$)-Arg($X^6$)-Gln-Gln-Gly-Glu($X^3$)-Arg($X^6$)-Asn-Gln-Glu($X^3$)-Gln-Gly-Ala-Arg($X^6$)-Val-Arg($X^6$)-Leu-$X^8$

wherein $X^1$ is BOC, $X^2$ is 2,6-dichlorobenzyl, $X^3$ is benzyl ester, $X^4$ is Bzl, $X^5$ is Bzl, $X^6$ is Tos, $X^7$ is 2Cl-Z and $X^8$ is —O—CH$_2$-benzene-polystyrene resin support.

After the final Tyr residue has been coupled to the resin, the BOC group is removed with 45% TFA in CH$_2$Cl$_2$. In order to cleave and deprotect the remaining protected peptide-resin, it is treated with 1.5 ml. anisole, 0.25 ml. methylethylsulfide and 10 ml. hydrogen fluoride (HF) per gram of peptide-resin, at −20°C. for one-half hour and at 0.°C. for one-half hour. After elimination of the HF under high vacuum, the resin-peptide remainder is washed alternately with dry diethyl ether and chloroform, and the peptide is then extracted with degassed 2N aqueous acetic acid. Lyophilization of the acetic acid extract provides a white fluffy material.

The cleaved and deprotected peptide is then dissolved in 30% acetic acid and subjected to Sephadex G-50 fine gel filtration.

The peptide is then further purified by CM-32 carboxymethyl cellulose (Whatman) cation-exchange chromatography (1.8 × 18 cm., $V_{bed}$ = 50 ml.) using a concave gradient generated by dropping 1 L. of 0.4 M NH$_4$OAc, pH 6.5 into a mixing flask containing 400 ml. 0.01. M. NH$_4$OAc, pH 4.5. Final purification is carried out using partition chromatography on Sephadex G-50 fine support (Pharmacia) with a nBuOH:EtOH:pyridine:0.2% N HOAc (4:1:1:7) solvent system. Purification details are generally set forth in Ling et al. *Biochem. Biophys. Res. Commun.* 95, 945 (1980). The chromatographic fractions are carefully monitored by TLC, and only the fractions showing substantial purity are pooled.

The synthesis is repeated using an MBHA resin to produce the same peptide having an amidated C-terminus, generally following the procedure described in U.S. Patent No. 4,292,313 to link Leu to the MBHA resin.

## Example II

To determine the effectiveness of the peptide to promote the release of growth hormone, *in vitro* assays are carried out using synthetic hGRF(1—44)-NH$_2$ in side-by-side comparison with pGRF(1—44)-NH$_2$ and of a GRF Reference Standard having a known effectiveness to promote the release of growth hormone from pituitary cells. The GRF Reference standard is described and defined in Brazeau, et al., *Endocrinology*, Vol. 110 A538 (1982) and is an amount of a preparation of rat hypothalamic origin that produces a half-maximal response in terms of GH release in a pituitary cell monolayer bioassay. Cultures are used which include cells of rat pituitary glands removed some four to five days previously. Both cultures of a defined standard medium and cultures which are considered optimal for the secretion of growth hormone are used for the comparative testing, in the general manner described in Brazeau, et al. *Regulatory Peptides*, 1, 255, 1981. Incubation with the substance to be tested is carried out for 3 to 4 hours, and aliquots of the culture medium are removed and processed to measure their contents in immunoreactive GH(ir GH) by a well-characterized radioimmunoassay.

The results of this comparative testing shows that, in equimolar ratios, pGRF(1—44)-NH$_2$ has the full intrinsic biological activity of the synthetic hGRF peptide and close to the same potency.

## Example III

The synthesis of bGRF(1—44) amide having the formula:

H-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Asn-Arg-Gln-Gln-Gly-Glu-Arg-Asn-Gln-Glu-Gln-Gly-Ala-Lys-Val-Arg-Leu-NH$_2$

is conducted in a stepwise manner using a Beckman 990 Peptide Synthesizer and an MBHA resin. Coupling of BOC-Leu to the resin is performed by the general procedure set forth in U.S. Patent No. 4,292,313, and it results in the substitution of about 0.2—0.6 mmol Leu per gram of resin depending on the substitution of the MBHA resin used. All solvents that are used are carefully degassed by sparging with an inert gas, preferably helium, to insure the absence of oxygen that might undesirably oxidize the sulfur of the Met residue.

After deprotection and neutralization, the peptide chain is built step-by-step on the resin. Deprotection, neutralization and addition of each amino acid is performed in general accordance with the procedure set forth in detail in Guillemin et al. U.S. Patent No. 3,904,594. The couplings are specifically carried out as set out in Schedule A of Example I.

The coupling reactions are carried out as described in Example I, and at the end of the synthesis, the following composition is obtained:

$X^1$-Tyr($X^2$)-Ala-Asp($X^3$)-Ala-Ile-Phe-Thr($X^4$)-Asn-Ser($X^5$)-Tyr($X^2$)-Arg($X^6$)-Lys($X^7$)-Val-Leu-Gly-Gln-Leu-Ser($X^5$)-Ala-Arg($X^6$)-Lys($X^7$)-Leu-Leu-Gln-Asp($X^3$)-Ile-Met-Asn-Arg($X^6$)-Gln-Gln-Gly-Glu($X^3$)-Arg($X^6$)-Asn-Gln-Glu($X^3$)-Gln-Gly-Ala-Lys($X^7$)-Val-Arg($X^6$)-Leu-$X^8$

wherein $X^1$ is BOC, $X^2$ is 2,6-dichlorobenzyl, $X^3$ is benzyl ester, $X^4$ is Bzl, $X^5$ is Bzl, $X^6$ is Tos, $X^7$ is 2Cl-Z and $X^8$ is —NH—MBHA resin support.

After the final Tyr residue has been coupled to the resin, the BOC group is removed with 45% TFA in $CH_2Cl_2$. In order to cleave and deprotect the remaining protected peptide-resin, it is treated with 1.5 ml. anisole, 0.25 ml. methylethylsulfide and 10 ml. hydrogen fluoride (HF) per gram of peptide-resin, at −20°C. for one-half hour and at 0°C for one-half hour. After elimination of the HF under high vacuum, the resin-peptide remainder is washed alternately with dry diethyl ether and chloroform, and the peptide is then extracted with degassed 2N aqueous acetic acid. Lyophilization of the acetic acid extract provides a white fluffy material.

The cleaved and deprotected peptide is then dissolved in 30% acetic acid and subjected to Sephadex G-50 fine gel filtration.

The peptide is then further purified using cation-exchange chromatography followed by partition chromatography as set forth in Example I.

The synthesis is repeated using a chloromethylated resin to produce the same peptide having a free acid C-terminus, generally following the procedure described in *Biopolymers, 12,* 2513—19 (1973) to link Leu to the chloromethylated resin.

The synthesis is repeated using a chloromethylated resin to produce the same peptide having a free acid C-terminus using the procedure of Example I.

## Example IV

To determine the effectiveness of the peptide to promote the release of growth hormone, *in vito* assays are carried out using synthetic hGRF(1—44)-$NH_2$ in side-by-side comparison with equimolar concentrations of bGRF(1—44)-$NH_2$, as set forth hereinbefore in Example II.

The results of this comparative testing shows that, in equimolar ratios, bGRF(1—44)-$NH_2$ has the full intrinsic biological activity of the synthetic peptide and close to the same potency. In multiple doses factorial design experiments, bGRF is shown to have the same intrinsic activity as hGRF(1—44)-$NH_2$ and a specific activity equal to about 70% of hGRF(1—44)-$NH_2$ with confidence limits of 54—93%.

## Example V

The synthesis of oGRF(1—44) amide having the formula:

H-Tyr-Ala-Asp-Ala-Ile-Phe-Thr-Asn-Ser-Tyr-Arg-Lys-Ile-Leu-Gly-Gln-Leu-Ser-Ala-Arg-Lys-Leu-Leu-Gln-Asp-Ile-Met-Asn-Arg-Gln-Gln-Gly-Glu-Arg-Asn-Gln-Glu-Gln-Gly-Ala-Lys-Val-Arg-Leu-$NH_2$

is conducted in a stepwise manner using a Beckman 990 Peptide Synthesizer and an MBHA resin, as set forth in Example III. At the end of the synthesis, the following composition is obtained:

$X^1$-Tyr($X^2$)-Ala-Asp($X^3$)-Ala-Ile-Phe-Thr($X^4$)-Asn-Ser($X^5$)-Tyr($X^2$)-Arg($X^6$)-Lys($X^7$)-Ile-Leu-Gly-Gln-Leu-Ser($X^5$)-Ala-Arg($X^6$)-Lys($X^7$)-Leu-Leu-Gln-Asp($X^3$)-Ile-Met-Asn-Arg($X^6$)-Gln-Gln-Gly-Glu($X^3$)-Arg($X^6$)-Asn-Gln-Glu($X^3$)-Gln-Gly-Ala-Lys($X^7$)-Val-Arg($X^6$)-Leu-$X^8$

wherein $X^1$ is BOC, $X^2$ is 2,6-dichlorobenzyl, $X^3$ is benzyl ester, $X^4$ is Bzl, $X^5$ is Bzl, $X^6$ is Tos, $X^7$ is 2Cl-Z and $X^8$ is —NH—MBHA resin support.

After the final Tyr residue has been coupled to the resin, the BOC group is removed with 45% TFA in $CH_2Cl_2$. Cleavage and deprotection of the remaining protected peptide-resin is carried out as specified in Example III. The cleaved and deprotected peptide is dissolved in 30% acetic acid, subjected to Sephadex G-50 fine gel filtration, cation-exchange chromatography and finally to partition chromatography as set forth in Example I.

The synthesis is repeated using a chloromethylated resin to produce the same peptide having a free acid C-terminus following the procedure described in Example I.

## Example VI

To determine the effectiveness of the peptide to promote the release of growth hormone, *in vitro* assays are carried out using synthetic hGRF(1—44)-$NH_2$ in side-by-side comparison with equimolar concentrations of the synthetic oGRF(1—44)-$NH_2$ of Example V as set forth hereinbefore in Example II.

The results of this comparative testing shows that, in equimolar ratios, the oGRF(1—44) has the full intrinsic biological activity of the synthetic peptide and close to the same potency. In multiple doses factorial design experiments, oGRF is shown to have about the same intrinsic activity as hGRF(1—44)-$NH_2$

and a specific activity equal to about 70% of hGRF(1—44)-NH$_2$.

Chronic administration of synthetic pGRF, bGRF and oGRF peptides to farm animals, particularly hogs, cattle, goats and sheep, or other warm-blooded animals is expected to promote anabolism and thus increase body weight in terms of muscle mass. The use in veterinary medicine of the GRF of its species, i.e., oGRF in sheep, bGRF in cattle or goats, is the ideal situation since the moelcule injected or otherwise administered will not be antigenic, being of the same species as that of the animal treated. It should also increase milk production in the female of the species. Use in aquiculture for raising fish and other cold-blooded marine animals to accelerate growth may also be important. Administration to animals at a purity as low as about 5% may be acceptable and will generally be carried out using a combination of the peptide and a veterinarily acceptable solid or liquid carrier to form what for purposes of this application is broadly termed a pharmaceutical composition.

Synthetic GRF or the nontoxic salts thereof, combined with a pharmaceutically acceptable carrier to form a pharmaceutical composition, may be administered to mammals, including humans, either intravenously, subcutaneously, intramuscularly, intranasally or orally. The administration may be employed by a physician to stimulate the release of growth hormone where the host being treated requires such therapeutic treatment. The required dosage will vary with the particular condition being treated, with the severity of the condition and with the duration of desired treatment.

Such peptides are often adminstered in the form of pharmaceutically acceptable nontoxic salts, such as acid addition salts or metal complexes, e.g. with zinc, iron or the like (which are considered as salts for purposes of this application). Illustrative of such acid addition salts are hydrochloride, hydrobromide, sulphate, phosphate, maleate, acetate, citrate, benzoate, succinate, malate, ascorbate, tartrate and the like. If the active ingredient is to be administered in tablet form, the tablet may contain a binder, such as tragacanth, corn starch or gelatin; a disintegrating agent, such as alginic acid; and a lubricant, such as magnesium stearate. If administration in liquid form is desired, sweetening and/or flavoring may be used, and intravenous administration in isotonic saline, phosphate buffer solutions or the like may be effected.

The peptides should be administered under the guidance of a physician, and pharmaceutical compositions will usually contain the peptide in conjuction with a conventional, pharmaceutically-acceptable carrier. Usually, the dosage will be from about 20 to about 2000 nanograms of the peptide per kilogram of the body weight of the host.

Although the invention has been described with regard to its preferred embodiments, modifications in the 44-member chain, particularly deletions beginning at the carboxyl terminal of the peptide, can be made in accordance with the known experimental evidence previously obtained with hGRF and following the practices to date to create fragments 34 to 43 residues in length, e.g. pGRF(1—34), bGRF(1—35), oGRF(1—40) and oGRF(1—37), or even shorter fragments, i.e. oGRF(1—32) bGRF(1—29) and oGRF(1—27), which fragments may have either NH$_2$ or OH at the C-terminal, that retain the intrinsic biological activity of the peptide. Moreover, additions can be made to either terminal, or to both terminals, and/or generally equivalent residues can be substituted for naturally occurring residues, as is well-known in the overall art of peptide chemistry to produce analogs having at least a substantial portion of the potency of the native polypeptide.

**Claims for the Contracting States: BE CH DE FR GB IT LI LU NL SE**

1. A synthetic peptide having the sequence:

Tyr-Ala-Asp-Ala-Ile-Phe-Thr-Asn-Ser-Tyr-Arg-Lys-R$_{13}$-Leu-Gly-Gln-Leu-Ser-Ala-Arg-Lys-Leu-Leu-Gln-Asp-Ile-Met-R$_{28}$-Arg-Gln-Gln-Gly-Glu-Arg-Asn-Gln-Glu-Gln-Gly-Ala-R$_{41}$-Val-Arg-Leu

wherein R$_{13}$ is Val or Ile; R$_{28}$ is Ser or Asn and R$_{41}$ is Arg or Lys or a biologically active fragment thereof, or a nontoxic salt thereof.

2. The peptide of Claim 1 wherein R$_{28}$ is Asn and R$_{41}$ is Lys.

3. The peptide of Claim 2 wherein R$_{13}$ is Ile.

4. The peptide of Claim 1 wherein R$_{28}$ is Ser and R$_{41}$ is Arg.

5. The peptide of Claim 2 or 4 wherein R$_{13}$ is Val.

6. A synthetic peptide having the formula of any one of Claims 1—5 wherein the C-terminal is amidated.

7. The peptide of Claim 1 having the formula

H-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-Gln-Gln-Gly-Glu-Arg-Asn-Gln-Glu-Gln-Gly-Ala-Arg-Val-Arg-Leu-NH$_2$

or a biologically active fragment thereof extending from the N-terminus to at least residue-34.

8. The peptide of Claim 1 having the formula

H-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Asn-Arg-Gln-Gln-Gly-Glu-Arg-Asn-Gln-Glu-Gln-Gly-Ala-Lys-Val-Arg-Leu-NH$_2$

or a biologically active fragment thereof extending from the N-terminus to at least residue-28.

9. The peptide of Claim 1 having the formula

H-Tyr-Ala-Asp-Ala-Ile-Phe-Thr-Asn-Ser-Tyr-Arg-Lys-Ile-Leu-Gly-Gln-Leu-Ser-Ala-Arg-Lys-Leu-Leu-Gln-Asp-Ile-Met-Asn-Arg-Gln-Gln-Gly-Glu-Arg-Asn-Gln-Glu-Gln-Gly-Ala-Lys-Val-Arg-Leu-NH$_2$

or a biologically active fragment thereof extending from the N-terminus to at least residue-28.

10. A composition for accelerating the growth of warm-blooded non-human animals comprising a peptide as defined in Claim 1 and a veterinarily acceptable solid or liquid carrier therefor.

11. A composition for use in accelerating the growth and/or milk production of bovine or caprine animals comprising a synthetic peptide in accordance with Claim 8 in combination with a veterinarily acceptable carrier therefor.

12. A composition for use in accelerating the growth of porcine animals comprising a synthetic peptide in accordance with Claim 7 in combination with a veterinarily acceptable carrier therefor.

13. A composition for use in accelerating the growth and/or milk production of ovine animals comprising a synthetic peptide in accordance with Claim 9 in combination with a veterinarily acceptable carrier therefor.

14. A composition in accordance with Claim 1 for use in aquiculture to accelerate the growth of cold-blooded animals.

**Claims for the Contracting State: AT**

1. A process for the manufacture of compounds defined by the formula (I):

H-Tyr-Ala-Asp-Ala-Ile-Phe-Thr-Asn-Ser-Tyr-Arg-Lys-R$_{13}$-Leu-Gly-Gln-Leu-Ser-Ala-Arg-Lys-Leu-Leu-Gln-Asp-Ile-Met-R$_{28}$-Arg-Gln-Gln-Gly-Glu-Arg-Asn-Gln-Glu-Gln-Gly-Ala-R$_{41}$-Val-Arg-Leu-Y

wherein R$_{13}$ is Val or Ile; R$_{28}$ is Ser or Asn; R$_{41}$ is Arg or Lys; and Y is OH or NH$_2$ comprising (a) forming a compound having at least one protective group and the formula (II):

X$^1$-Tyr(X$^2$)-Ala-Asp(X$^3$)-Ala-Ile-Phe-Thr(X$^4$)-Asn-Ser(X$^5$)-Tyr(X$^2$)-Arg(X$^6$)-Lys(X$^7$)-R$_{13}$-Leu-Gly-Gln-Leu-Ser(X$^5$)-Ala-Arg(X$^6$)-Lys(X$^7$)-Leu-Leu-Gln-Asp(X$^3$)-Ile-Met-R$_{28}$(X$^5$)-Arg(X$^6$)-Gln-Gln-Gly-Glu(X$^3$)-Arg(X$^6$)-Asn-Gln-Glu(X$^3$)-Gln-Gly-Ala-R$_{41}$(X$^6$ or X$^7$)-Val-Arg(X$^6$)-Leu-X$^8$

wherein any or all of the residues from R$_{28}$ through the C-terminus can be omitted, wherein (X$^1$), (X$^2$), (X$^3$), (X$^4$), (X$^5$), (X$^6$) and (X$^7$) are each either hydrogen or a protective group and wherein (X$^8$) is either amide or hydroxyl or a protective group, and (b) splitting off the protective group or groups from said compound of the formula (II) to provide a peptide having the formula (I) or a biologically active fragment thereof and, if desired, (c) converting the resulting peptide into a nontoxic addition salt thereof.

2. A process in accordance with Claim 1 wherein R$_{28}$ is Asn and R$_{41}$ is Lys.

3. A process in accordance with Claim 2 wherein R$_{13}$ is Ile.

4. A process in accordance with Claim 2 wherein R$_{13}$ is Val.

5. A process in accordance with Claim 1 wherein R$_{13}$ is Val, R$_{28}$ is Ser and R$_{41}$ is Arg.

6. A process in accordance with any one of Claims 1 to 5 wherein Y is NH$_2$.

7. A process in accordance with any one of Claims 1 to 5 wherein Y is OH.

8. A process in accordance with any one of Claims 1 to 7 wherein X$^1$ is hydrogen or an α-amino protecting group; X$^2$ is hydrogen or a protecting group for the phenolic hydroxyl group of Tyr; X$^3$ is hydrogen or a protecting group for the carboxyl group Asp or Glu; X$^4$ is hydrogen or a protecting group for the alcoholic hydroxyl group of Thr; X$^5$ is hydrogen or a protecting group for the alcoholic hydroxyl group of Ser; X$^6$ is hydrogen or a protecting group for the guanidino group of Arg; X$^7$ is hydrogen or a protecting group for the side chain amino group of Lys; and X$^8$ is selected from the class consisting of OH, OCH$_3$, esters, amides, hydrazides, —O—CH$_2$-resin support and —NH-resin support.

9. A process in accordance with Claim 8 wherein X$^1$ is BOC, X$^2$ is 2,6-dichlorobenzyl, X$^3$ is Bzl, X$^4$ is Bzl, X$^5$ is Bzl, X$^6$ is Tos, X$^7$ is 2-chlorobenzyloxycarbonyl and X$^8$ is —O—CH$_2$-resin support.

10. A process in accordance with Claim 8 wherein X$^1$ is BOC, X$^2$ is 2,6-dichlorobenzyl, X$^3$ is Bzl, X$^4$ is Bzl, X$^5$ is Bzl, X$^6$ is Tos, X$^7$ is 2-chlorobenzyloxycarbonyl and X$^8$ is —NH-resin support.

11. A method of improving agriculture wherein release of GH in warm-blooded non-human animals is stimulated to promote increase in muscle mass and/or milk production by administering an effective amount of a peptide or salt as defined in Claim 1.

12. A method of improving agriculture wherein release of GH in cold-blooded animals is stimulated to accelerate the growth thereof by administering a peptide or salt as defined in Claim 1.

**0 137 689**

1. Synthetisches Peptid mit der Sequenz:

Tyr-Ala-Asp-Ala-Ile-Phe-Thr-Asn-Ser-Tyr-Arg-Lys-$R_{13}$-Leu-Gly-Gln-Leu-Ser-Ala-Arg-Lys-Leu-Leu-Gln-Asp-Ile-Met-$R_{28}$-Arg-Gln-Gln-Gly-Glu-Arg-Asn-Gln-Glu-Gln-Gly-Ala-$R_{41}$-Val-Arg-Leu,

wobei $R_{13}$ Val oder Ile bedeutet; $R_{28}$ Ser oder Asn bedeutet und $R_{41}$ Arg oder Lys oder ein biologisch aktives Fragment dessen bedeutet, oder ein nicht-toxisches Salz dessen.

2. Peptid nach Anspruch 1, dadurch gekennzeichnet, daß $R_{28}$ Asn bedeutet und $R_{41}$ Lys bedeutet.

3. Peptid nach Anspruch 2, dadurch gekennzeichnet, daß $R_{13}$ Ile bedeutet.

4. Peptid nach Anspruch 1, dadurch gekennzeichnet, daß $R_{28}$ Ser bedeutet und $R_{41}$ Arg bedeutet.

5. Peptid nach Anspruch 2 oder 4, dadurch gekennzeichnet, daß $R_{13}$ Val bedeutet.

6. Synthetisches Peptid mit der Formel eines der Ansprüche 1—5, dadurch gekennzeichnet, daß der C-Terminus amidiert ist.

7. Peptid nach Anspruch 1 mit der Formel

H-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-Gln-Gln-Gly-Glu-Arg-Asn-Gln-Glu-Gln-Gly-Ala-Arg-Val-Arg-Leu-$NH_2$

oder ein biologisch aktives Fragment dessen, welches sich vom N-Terminus bis mindestens zum Rest 34 erstreckt.

8. Peptid nach Anspruch 1 mit der Formel

H-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Asn-Arg-Gln-Gln-Gly-Glu-Arg-Asn-Gln-Glu-Gln-Gly-Ala-Lys-Val-Arg-Leu-$NH_2$

oder ein biologisch aktives Fragment dessen, welches sich vom N-Terminus bis mindestens zum Rest 28 erstreckt.

9. Peptid nach Anspruch 1 mit der Formel

H-Tyr-Ala-Asp-Ala-Ile-Phe-Thr-Asn-Ser-Tyr-Arg-Lys-Ile-Leu-Gly-Gln-Leu-Ser-Ala-Arg-Lys-Leu-Leu-Gln-Asp-Ile-Met-Asn-Arg-Gln-Gln-Gly-Glu-Arg-Asn-Gln-Glu-Gln-Gly-Ala-Lys-Val-Arg-Leu-$NH_2$

oder ein biologisch aktives Fragment dessen, welches sich vom N-Terminus bis mindestens zum Rest 28 erstreckt.

10. Zusammensetzung zur Beschleunigung des Wachstums von nicht-menschlichen warmblütigen Tieren, die ein Peptid nach Anspruch 1 und einen tierärztlich annehmbaren festen oder flüssigen Träger dessen enthält.

11. Zusammensetzung zur Verwendung bei der Beschleunigung des Wachstums und/oder der Milchproduktion von Rindern und Ziegen, die ein synthetisches Peptid nach Anspruch 8 in Verbindung mit einem tierärztlich annehmbaren Träger dessen enthält.

12. Zusammensetzung zur Verwendung bei der Beschleunigung des Wachstums von Schweinen, die ein synthetisches Peptid nach Anspruch 7 in Verbindung mit einem tierärztlich annehmbaren Träger dessen enthält.

13. Zusammensetzung zur Verwendung bei der Beschleunigung des Wachstums und/oder der Milchproduktion von Rindern, die ein synthetisches Peptid nach Anspruch 9 in Verbindung mit einem tierärztlich annehmbaren Träger dessen enthält.

14. Zusammensetzung nach Anspruch 1 zur Verwendung in der Aquikultur zur Beschleunigung des Wachstums von Kaltblütern.

**Patentansprüche für den Vertragsstaat: AT**

1. Verfahren zur Herstellung einer Verbindung, die durch die Formel (I) definiert ist:

H-Tyr-Ala-Asp-Ala-Ile-Phe-Thr-Asn-Ser-Tyr-Arg-Lys-$R_{13}$-Leu-Gly-Gln-Leu-Ser-Ala-Arg-Lys-Leu-Leu-Gln-Asp-Ile-Met-$R_{28}$-Arg-Gln-Gln-Gly-Glu-Arg-Asn-Gln-Glu-Gln-Gly-Ala-$R_{41}$-Val-Arg-Leu-Y

wobei $R_{13}$ Val oder Ile bedeutet, $R_{28}$ Ser oder Asn bedeutet; $R_{41}$ Arg oder Lys bedeutet; und Y OH oder $NH_2$ bedeutet, welches (a) die Bildung einer Verbindung mit mindestens einer Schutzgruppe und der Formel (II) umfaßt:

$X^1$-Tyr($X^2$)-Ala-Asp($X^3$)-Ala-Ile-Phe-Thr($X^4$)-Asn-Ser($X^5$)-Tyr($X^2$)-Arg($X^6$)-Lys($X^7$)-$R_{13}$-Leu-Gly-Gln-Leu-Ser($X^5$)-Ala-Arg($X^6$)-Lys($X^7$)-Leu-Leu-Gln-Asp($X^3$)-Ile-Met-$R_{28}$($X^5$)-Arg($X^6$)-Gln-Gln-Gly-Glu($X^3$)-Arg($X^6$)-Asn-Gln-Glu($X^3$)-Gln-Gly-Ala-$R_{41}$($X^6$ oder $X^7$)-Val-Arg($X^6$)-Leu-$X^8$

wobei ein oder alle Reste von $R_{28}$ bis zum C-Terminus weggelassen werden können, wobei $(X^1)$, $(X^2)$, $(X^3)$, $(X^4)$, $(X^5)$, $(X^6)$ und $(X^7)$ jedes entweder Wasserstoff oder eine Schutzgruppe bedeutet und wobei $(X^8)$ entweder ein Amid oder ein Hydroxyl oder eine Schutzgruppe bedeutet, und (b) die Abspaltung der Schutzgruppe oder -gruppen von der besagten Verbindung der Formel (II) umfaßt, um ein Peptid der Formel (I) oder ein biologisch aktives Fragment dessen zu erhalten, und, falls dies gewünscht ist, (c) die Überführung des erhaltenen Peptids in ein nicht-toxisches Additionssalz dessen umfaßt.

2. Verfahren nach Anspruch 1, dadurch gekennzeichnet, daß $R_{28}$ Asn bedeutet und $R_{41}$ Lys bedeutet.

3. Verfahren nach Anspruch 2, dadurch gekennzeichnet, daß $R_{13}$ Ile bedeutet.

4. Verfahren nach Anspruch 2, dadurch gekennzeichnet, daß $R_{13}$ Val bedeutet.

5. Verfahren nach Anspruch 1, dadurch gekennzeichnet, daß $R_{13}$ Val bedeutet, $R_{28}$ Ser bedeutet und $R_{41}$ Arg bedeutet.

6. Verfahren nach einem der Ansprüche 1 bis 5, dadurch gekennzeichnet, daß Y $NH_2$ bedeutet.

7. Verfahren nach einem der Ansprüche 1 bis 5, dadurch gekennzeichnet, daß Y OH bedeutet.

8. Verfahren nach einem der Ansprüche 1 bis 7, dadurch gekennzeichnet, daß $X^1$ Wasserstoff oder eine α-Amino-Schutzgruppe bedeutet; $X^2$ bedeutet Wasserstoff oder eine Schutzgruppe für die phenolische Hydroxylgruppe von Tyr; $X^3$ bedeutet Wasserstoff oder eine Schutzgruppe für die Carboxylgruppe von Asp oder Glu; $X^4$ bedeutet Wasserstoff oder eine Schutzgruppe für die alkoholische Hydroxylgruppe von Thr; $X^5$ bedeutet Wasserstoff oder eine Schutzgruppe für die alkoholische Hydroxylgruppe von Ser; $X^6$ bedeutet Wasserstoff oder eine Schutzgruppe für die Guanidinogruppe von Arg; $X^7$ bedeutet Wasserstoff oder eine Schutzgruppe für die Seitenketten-Aminogruppe von Lys; und $X^8$ wird ausgewählt aus der Klasse, die aus OH, $OCH_3$, Estern, Amiden, Hydraziden, $—O—CH_2$-Harzträgern und NH-Harzträgern besteht.

9. Verfahren nach Anspruch 8, dadurch gekennzeichnet, daß $X^1$ BOC bedeutet, $X^2$ 2,6-Dichlorbenzyl bedeutet, $X^3$ Bzl bedeutet, $X^4$ Bzl bedeutet, $X^5$ Bzl bedeutet, $X^6$ Tos bedeutet, $X^7$ 2-Chlorbenzyloxycarbonyl bedeutet und $X^8$ ein $—O—CH_2—$Harzträger ist.

10. Verfahren nach Anspruch 8, dadurch gekennzeichnet, daß $X^1$ BOC bedeutet, $X^2$ 2,6-Dichlorbenzyl bedeutet, $X^3$ Bzl bedeutet, $X^4$ Bzl bedeutet, $X^5$ Bzl bedeutet, $X^6$ Tos bedeutet, $X^7$ 2-Chlorbenzyloxycarbonyl bedeutet und $X^8$ ein $—NH—$Harzträger ist.

11. Methode zur Verbesserung der Landwirtschaft, dadurch gekennzeichnet, daß die Freisetzung von GH bei nicht menschlichen warmblütigen Tieren stimuliert wird und zu einer Vergrößerung der Muskelmasse und/oder der Milchproduktion führt, durch die Verabreichung einer wirksamen Menge eines Peptids oder Salzes nach Anspruch 1.

12. Methode zur Verbesserung der Aquikultur, dadurch gekennzeichnet, daß die Freisetzung von GH bei Kaltblütern stimuliert wird und so deren Wachstum beschleunigt, durch die Verabreichung einer wirksamen Menge eines Peptids oder Salzes nach Anspruch 1.

**Revendications pour les Etats contractants: BE CH DE FR GB IT LI LU NL SE**

1. Un peptide synthétique ayant la séquence:

Tyr-Ala-Asp-Ala-Ile-Phe-Thr-Asn-Ser-Tyr-Arg-Lys-$R_{13}$-Leu-Gly-Gln-Leu-Ser-Ala-Arg-Lys-Leu-Leu-Gln-Asp-Ile-Met-$R_{28}$-Arg-Gln-Gln-Gly-Glu-Arg-Asn-Gln-Glu-Gln-Gly-Ala-$R_{41}$-Val-Arg-Leu,

dans lequel $R_{13}$ est Val ou Ile; $R_{28}$ est Ser ou Asn et $R_{41}$ est Arg ou Lys ou un fragment biologiquement actif de celui-ci, ou un sel non toxique de celui-ci.

2. Le peptide de la revendication 1 dans lequel $R_{28}$ est Asn et $R_{41}$ est Lys.

3. Le peptide de la revendication 2 dans lequel $R_{13}$ est Ile.

4. Le peptide de la revendication 1 dans lequel $R_{28}$ est Ser et $R_{41}$ est Arg.

5. Le peptide de la revendication 2 ou 4 dans lequel $R_{13}$ est Val.

6. Un peptide synthétique ayant la formule de l'une quelconque des revendications 1 à 5, dans lequel le C-terminal est amidifié.

7. Le peptide de la revendication 1 ayant la formule

H-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-Gln-Gln-Gly-Glu-Arg-Asn-Gln-Glu-Gln-Gly-Ala-Arg-Val-Arg-Leu-$NH_2$

ou un fragment biologiquement actif de celui-ci s'étendant du N-terminal à au moins le résidu-34.

8. Le peptide de la revendication 1 ayant la formule

H-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Asn-Arg-Gln-Gln-Gly-Glu-Arg-Asn-Gln-Glu-Gln-Gly-Ala-Lys-Val-Arg-Leu-$NH_2$

ou un fragment biologiquement actif de celui-ci s'étendant du N-terminal à au moins le résidu-28.

9. Le peptide de la revendication 1 ayant la formule

## 0 137 689

H-Tyr-Ala-Asp-Ala-Ile-Phe-Thr-Asn-Ser-Tyr-Arg-Lys-Ile-Leu-Gly-Gln-Leu-Ser-Ala-Arg-Lys-Leu-Leu-Gln-Asp-Ile-Met-Asn-Arg-Gln-Gln-Gly-Glu-Arg-Asn-Gln-Glu-Gln-Gly-Ala-Lys-Val-Arg-Leu-NH$_2$

ou un fragment biologiquement actif de celui-ci s'étendant du N-terminal à au moins le résidu-28.

10. Une composition pour accélérer la croissance des animaux à sang chaud non-humains comprenant un peptide comme définit dans la revendication 1 et un véhicule solide ou liquide de celui-ci, vétérinairement acceptable.

11. Une composition à utiliser pour accélérer la croissance et/ou la production de lait d'animaux bovins ou caprins, comprenant un peptide synthétique selon la revendication 8 en combinaison avec un véhicule de celui-ci, vétérinairement acceptable.

12. Une composition à utiliser pour accélérer la croissance des animaux porcins, comprenant un peptide synthétique selon la revendication 7 en combinaison avec un véhicule de celui-ci, vétérinairement acceptable.

13. Une composition à utiliser pour accélérer la croissance et/ou la production de lait des animaux ovins, comprenant un peptide synthétique selon la revendication 9 en combinaison avec un véhicule de celui-si, vétérinairement acceptable.

14. Une composition selon la revendication 1 pour l'utilisation en aquiculture pour accélérer la croissance des animaux à sang froid.

**Revendications pour l'Etat contractant: AT**

1. Un procédé de fabrication des composés définis par la formule (I):

H-Tyr-Ala-Asp-Ala-Ile-Phe-Thr-Asn-Ser-Tyr-Arg-Lys-R$_{13}$-Leu-Gly-Gln-Leu-Ser-Ala-Arg-Lys-Leu-Leu-Gln-Asp-Ile-Met-R$_{28}$-Arg-Gln-Gln-Gly-Glu-Arg-Asn-Gln-Glu-Gln-Gly-Ala-R$_{41}$-Val-Arg-Leu-Y

dans lequel R$_{13}$ est Val ou Ile; R$_{28}$ est Ser ou Asn, R$_{41}$ est Arg ou Lys; et Y est OH ou NH$_2$ comprenant (a) la formation d'un composé ayant au moins un groupe protecteur et la formule (II):

X$^1$-Tyr(X$^2$)-Ala-Asp(X$^3$)-Ala-Ile-Phe-Thr(X$^4$)-Asn-Ser(X$^5$)-Tyr(X$^2$)-Arg(X$^6$)-Lys(X$^7$)-R$_{13}$-Leu-Gly-Gln-Leu-Ser(X$^5$)-Ala-Arg(X$^6$)-Lys(X$^7$)-Leu-Leu-Gln-Asp(X$^3$)-Ile-Met-R$_{28}$(X$^5$)-Arg(X$^6$)-Gln-Gln-Gly-Glu(X$^3$)-Arg(X$^6$)-Asn-Gln-Glu(X$^3$)-Gln-Gly-Ala-R$_{41}$(X$^6$ ou X$^7$)-Val-Arg(X$^6$)-Leu-X$^8$

dans lequel un quelconque ou tous les résidus de R$_{28}$ au C-terminal peuvent être supprimés, dans laquelle (X$^1$), (X$^2$), (X$^3$), (X$^4$), (X$^5$), (X$^6$) et (X$^7$) sont chacun soit l'hydrogène, soit un groupe protecteur et dans lequel (X$^8$) est soit un amide, soit un hydroxyle, soit un groupe protecteur, et (b) l'élimination du ou des groupes protecteurs dudit composé de formule (II) pour donner un peptide ayant la formule (I) ou un fragment biologiquement actif de celui-ci et, si souhaité, (c) la transformation de peptide obtenu dans un sel d'addition non toxique, de celui-ci.

2. Un procédé selon la revendication 1, dans lequel R$_{28}$ est Asn et R$_{41}$ est Lys.

3. Un procédé selon la revendication 2, dans lequel R$_{13}$ est Ile.

4. Un procédé selon la revendication 2, dans lequel R$_{13}$ est Val.

5. Un procédé selon la revendication 1, dans lequel R$_{13}$ est Val, R$_{28}$ est Ser et R$_{41}$ est Arg.

6. Un procédé selon l'une quelconque des revendications 1 à 5, dans lequel Y est NH$_2$.

7. Un procédé selon l'une quelconque des revendications 1 à 5, dans lequel Y est OH.

8. Un procédé selon l'une quelconque des revendications 1 à 7, dans lequel X$^1$ est l'hydrogène ou un groupe protecteur d'un α-amino; X$^2$ est l'hydrogène ou un groupe protecteur du groupe hydroxyle phénolique de Tyr; X$^3$ est l'hydrogène ou un groupe protecteur du groupe carboxyle de Asp ou Glu; X$^4$ est l'hydrogène ou un groupe protecteur du groupe hydroxyle alcoolique de Thr; X$^5$ est l'hydrogène ou un groupe protecteur du groupe hydroxyle alcoolique de Ser; X$^6$ est l'hydrogène ou un groupe protecteur pour le groupe guanidino de Arg; X$^7$ est l'hydrogène ou un groupe protecteur pour le groupe amino de la chaîne latérale de Lys; et X$^8$ est choisi parmi la classe se composant de OH, OCH$_3$, des esters, des amides, des hydrazides, —O—CH$_2$-résine support et —NH-résine support.

9. Un procédé selon la revendication 8, dans lequel X$^1$ est BOC, X$^2$ est 2,6-dichlorobenzyle, X$^3$ est Bzl, X$^4$ est Bzl, X$^5$ est Bzl, X$^6$ est Tos, X$^7$ est 2-chlorobenzyloxycarbonyl et X$^8$ est —O—CH$_2$-résine support.

10. Un procédé selon la revendication 8, dans lequel X$^1$ est BOC, X$^2$ est 2,6-dichlorobenzyle, X$^3$ est Bzl, X$^4$ est Bzl, X$^5$ est Bzl, X$^6$ est Tos, X$^7$ est 2-chlorobenzyloxycarbonyl et X$^8$ est —NH-résine support.

11. Une méthode pour améliorer l'agriculture dans laquelle la libération de GH chez les animaux à sang chaud, non-humains, est stimulée pour favoriser l'augmentation de la masse musculaire et/ou la production de lait en administrant une quantité efficace d'un peptide ou sel comme définis dans la revendication 1.

12. Une méthode pour améliorer l'aquiculture dans laquelle la libération de GH chez les animaux à sang froid, est stimulée pour accélérer la croissance de ceux-ci en administrant une quantité efficace d'un peptide ou sel comme définis dans la revendication 1.

14